(19) Europäisches Patentamt
European Patent Office
Office européen des brevets

(11)  **EP 2 628 000 B1**

(12)  **FASCICULE DE BREVET EUROPEEN**

(45) Date de publication et mention
de la délivrance du brevet:
**16.11.2016  Bulletin 2016/46**

(51) Int Cl.:
***G01N 29/24*** *(2006.01)*  ***G01N 29/26*** *(2006.01)*
***G01N 29/265*** *(2006.01)*

(21) Numéro de dépôt: **11773801.3**

(22) Date de dépôt: **27.09.2011**

(86) Numéro de dépôt international:
**PCT/FR2011/052259**

(87) Numéro de publication internationale:
**WO 2012/049393 (19.04.2012 Gazette 2012/16)**

(54) **DISPOSITIF DE SONDAGE À ULTRASONS, PROCÉDÉ DE COMMANDE DE TRANSDUCTEURS D'UNE SONDE À ULTRASONS ET PROGRAMME D'ORDINATEUR CORRESPONDANT**

ULTRASCHALLSONDE, VERFAHREN ZUR STEUERUNG EINER ULTRASCHALLSONDE UND ENTSPRECHENDES COMPUTERPROGRAMM

ULTRASONIC PROBE, CONTROL METHOD FOR AN ULTRASONIC PROBE AND CORRESPONDING COMPUTER PROGRAM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.10.2010  FR 1058242**

(43) Date de publication de la demande:
**21.08.2013  Bulletin 2013/34**

(73) Titulaire: **Commissariat à l'Énergie Atomique et aux Énergies Alternatives**
**75015 Paris (FR)**

(72) Inventeurs:
 • **CASULA, Olivier**
  **F-91310 Longpont sur Orge (FR)**
 • **BEY, Sébastien**
  **F-91400 Orsay (FR)**

(74) Mandataire: **Bonnet, Michel**
  **Cabinet Bonnet**
  **93, rue Réaumur - Boîte 10**
  **75002 Paris (FR)**

(56) Documents cités:
  **FR-A1- 2 786 651     US-A- 5 161 413**

 • **TOULLELAN G ET AL: "APPLICATION OF A 3D SMART FLEXIBLE PHASED-ARRAY TO PIPING INSPECTION", AIP CONFERENCE PROCEEDINGS, vol. 975, 28 février 2008 (2008-02-28), pages 794-800, XP007918186, DOI: 10.1063/1.2902744**
 • **CASULA O ET AL: "ULTRASONIC NONDESTRUCTIVE TESTING OF COMPLEX COMPONENTS WITH FLEXIBLE PHASED-ARRAY TRANSDUCERS", PROCEEDINGS OF THE 10TH EUROPEAN CONFERENCE ON NON-DESTRUCTIVE TESTING, 1.3.25, 7 juin 2010 (2010-06-07), - 11 juin 2010 (2010-06-11), XP007918185,**

## Description

La présente invention concerne un dispositif de sondage à ultrasons, un procédé de commande de transducteurs d'une sonde à ultrasons et un programme d'ordinateur correspondant.

L'invention s'applique notamment au domaine de l'imagerie médicale, ainsi qu'au contrôle non destructif de pièces mécaniques, en particulier de pièces ayant une forme complexe.

La terminologie suivante sera utilisée dans la description qui va suivre, ainsi que dans les revendications.

Une onde ultrasonore, ou ultrasons, est une onde mécanique de fréquences supérieures à 20 000 Hertz se propageant dans un milieu solide, liquide ou gazeux.

Les termes « position ou orientation d'un élément X par rapport à un élément Y », signifient « position ou orientation d'un référentiel lié à l'élément X par rapport à un référentiel lié à l'élément Y ».

Le brevet français publié sous le numéro FR 2 786 651 décrit un dispositif de sondage à ultrasons comportant un boîtier, des moyens de localisation conçus pour fournir une position $P_{B/O}$ du boîtier par rapport à un référentiel lié à un objet à sonder, des moyens de détermination d'une loi de retards à partir de paramètres de focalisation, notés $P_{F/B}$, représentant une position souhaitée d'un point focal par rapport à un référentiel lié au boîtier, des moyens de commande conçus pour fournir des signaux de commande à partir de la loi de retards, et des transducteurs attachés au boîtier, conçus pour recevoir les signaux de commande et pour, en réponse, émettre dans l'objet des ondes ultrasonores retardées les unes par rapport aux autres suivant la loi de retards de manière qu'elles focalisent au point focal défini par les paramètres de focalisation $P_{F/B}$.

Dans ce document, les transducteurs sont attachés de manière mobile au boîtier afin de pouvoir épouser la géométrie de la surface de l'objet à sonder. Il est alors prévu des moyens pour déterminer les positions des transducteurs à chaque instant et pour fournir ces positions aux moyens de détermination de la loi de retards. Ainsi, les moyens de détermination de la loi de retards peuvent tenir compte des positions des transducteurs mobiles pour corriger la loi de retards qu'ils appliquent et conserver la position souhaitée du point focal par rapport au boîtier.

Mais cette souplesse a ses limites puisqu'il est prévu une unique position souhaitée du point focal par rapport au boîtier, prédéterminée pour toute la durée du sondage de l'objet et directement intégrée dans les formules appliquées par les moyens de détermination de la loi de retards.

Il peut ainsi être souhaité de prévoir un dispositif de sondage à ultrasons qui permette de présenter davantage de souplesse dans son utilisation, notamment par exemple en permettant des trajets complexes du point focal au cours du sondage de l'objet.

L'invention a donc pour objet un dispositif de sondage à ultrasons selon la revendication 1.

L'invention a également pour objet un procédé de commande de transducteurs d'une sonde à ultrasons selon la revendication 9.

L'invention a également pour objet un programme d'ordinateur selon la revendication 10.

L'invention sera mieux comprise à l'aide de la description qui va suivre, donnée uniquement à titre d'exemple et faite en se référant aux dessins annexés dans lesquels :

- la figure 1 représente schématiquement la structure générale d'un dispositif de sondage d'un objet comportant une sonde à ultrasons, selon un premier mode de réalisation de l'invention,
- la figure 2 illustre les étapes successives d'un procédé de sondage mis en oeuvre à l'aide du dispositif de la figure 1,
- la figure 3 illustre un trajet de la sonde de la figure 1 sur l'objet à sonder, ainsi que le déplacement d'un point focal de la sonde dans l'objet,
- la figure 4 représente schématiquement la structure générale d'un dispositif de sondage d'un objet comportant une sonde à ultrasons, selon un deuxième mode de réalisation de l'invention,
- la figure 5 illustre les étapes successives d'un procédé de sondage mis en oeuvre à l'aide du dispositif de la figure 4,
- la figure 6 illustre un trajet de la sonde de la figure 4 sur l'objet à sonder, ainsi que le déplacement d'un point focal de la sonde dans l'objet,
- la figure 7 représente schématiquement la structure générale d'un dispositif de sondage d'un objet comportant une sonde à ultrasons, selon un troisième mode de réalisation de l'invention, et
- la figure 8 illustre un trajet de la sonde de la figure 7 sur l'objet à sonder, ainsi que le déplacement d'un point focal de la sonde dans l'objet.

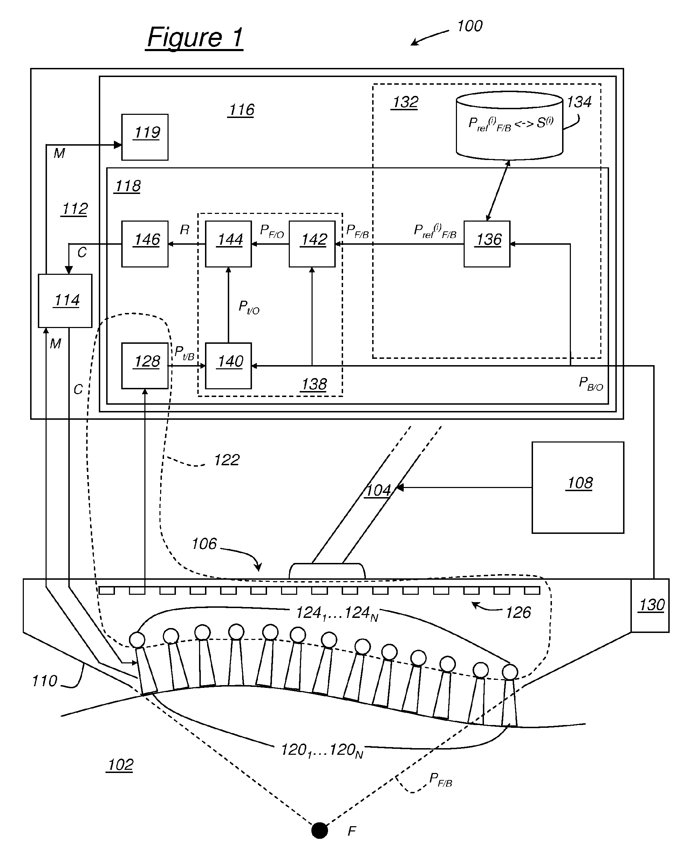

En référence à la figure 1, un dispositif de sondage 100 d'un objet 102 selon un premier mode de réalisation de l'invention comporte un bras 104, qui est articulé dans l'exemple décrit, une sonde à ultrasons 106 fixée au bras articulé 104 et des moyens 108 de commande du bras articulé 104 conçus pour commander le bras articulé 104 afin que ce dernier déplace la sonde 106 sur une surface de l'objet 102. Les moyens 108 de commande de bras articulé se basent généralement sur un trajet souhaité prédéfini de la sonde 106 par rapport à l'objet 102.

L'objet 102 est par exemple une pièce mécanique que l'on souhaite examiner par contrôle non destructif ou bien, dans un contexte médical, une partie du corps humain que l'on souhaite contrôler de manière non invasive.

En outre, le dispositif de sondage 100 comporte un circuit électronique 112, par exemple celui d'un ordinateur. Ce circuit électronique 112 est connecté à la sonde 106. Il présente une unité centrale de traitement 114, telle qu'un microprocesseur, et une mémoire 116 dans laquelle est enregistré un programme d'ordinateur 118 de commande de la sonde 106 et un programme d'ordinateur 119 de traitement de signaux. Ces programmes 118 et 119 comportent des instructions destinées à être exécutées par l'unité centrale de traitement 114 pour réaliser les fonctions définies par ces instructions.

La sonde 106 comporte tout d'abord un boîtier 110, c'est-à-dire un élément de structure indéformable qui sert de référentiel pour la sonde 106.

La sonde 106 comporte en outre des transducteurs $120_1...120_N$ attachés au boîtier 110 et conçus pour émettre des ondes ultrasonores dans l'objet 102 en réponse à des signaux de commande C fournis par le circuit électronique 112. Les transducteurs $120_1...120_N$ sont en outre conçus pour détecter des échos des ondes ultrasonores se réfléchissant sur et dans l'objet 102 et pour fournir des signaux de mesure M correspondant à ces échos, ces signaux de mesure M étant alors transmis au circuit électronique 112. C'est plus précisément l'unité centrale de traitement 114 qui est conçue pour émettre vers la sonde 106 les signaux de commande C et pour recevoir de la sonde 106 les signaux de mesure M.

Dans la sonde 106 de la figure 1, les transducteurs $120_1...120_N$ sont attachés de manière mobile au boîtier 110, de sorte qu'ils sont aptes à épouser la surface de l'objet 102 sur laquelle ils se déplacent, même lorsque cette dernière n'est pas plane, comme cela est illustré sur la figure 1.

En outre, le dispositif de sondage 100 comporte des moyens 122 de localisation des transducteurs $120_1...120_N$ conçus pour déterminer des positions des transducteurs $120_1...120_N$ par rapport au boîtier 110, ces positions étant notées $P_{t/B}$.

Dans le dispositif de sondage 100 de la figure 1, les moyens 122 de localisation des transducteurs $120_1...120_N$ comportent des émetteurs $124_1...124_N$ conçus pour émettre des ondes par exemple électromagnétiques. Chaque émetteur $124_1...124_N$ est fixé à un transducteur $120_1...120_N$ respectif. Les moyens 122 de localisation des transducteurs $120_1...120_N$ comportent en outre des récepteurs 126 fixés au boîtier 110 et conçus pour détecter les ondes émises par les émetteurs $124_1...124_N$. Les moyens 122 de localisation des transducteurs $120_1...120_N$ comportent en outre des instructions 128 du programme de commande 118 pour déterminer les positions $P_{t/B}$ des transducteurs $120_1...120_N$ à partir des détections des récepteurs 126, par exemple par triangulation. Les moyens de localisation 122 sont par exemple conformes à ceux décrits en détail dans le brevet FR 2 786 651.

En variante, d'autres moyens de localisation de transducteurs pourraient être utilisés, par exemples ceux également envisagés dans le brevet FR 2 786 651.

Le dispositif de sondage 100 comporte en outre des moyens 130 de localisation du boîtier 110 conçus pour fournir une position du boîtier 110 par rapport à l'objet 102, notée $P_{B/O}$. Dans le dispositif de sondage 100 de la figure 1, les moyens de localisation 130 comportent un capteur de position fixé au boîtier 110 et conçu pour déterminer la position réelle du boîtier 110 par rapport à l'objet 102. Selon une variante dans laquelle le dispositif de sondage 100 comporte un manche (non représenté) fixé au boîtier 110 à la place ou à l'extrémité du bras articulé 104, le capteur 130 peut être fixé au manche.

En variante, les moyens 130 de localisation du boîtier 110 pourraient être une base de données dans laquelle serait enregistré le trajet souhaité du boîtier 110 par rapport à l'objet 102, par exemple le trajet utilisé par les moyens 108 de commande du bras articulé 104. Dans ce cas, la position $P_{B/O}$ du boîtier 110 serait la position souhaitée du boîtier 110.

Le dispositif de sondage 100 comporte en outre des moyens 132 de mise à jour de paramètres de focalisation, notés $P_{F/B}$, représentant une position souhaitée d'un point focal F par rapport au boîtier 110. Les moyens de mise à jour 132 sont conçus pour mettre à jour les paramètres de focalisation $P_{F/B}$ à partir, d'une part, d'associations prédéfinies entre des positions sur le trajet souhaité du boîtier 110 et des valeurs correspondantes des paramètres de focalisation, et, d'autre part, de la position $P_{B/O}$ du boîtier 110.

Dans le dispositif de sondage de la figure 1, le trajet souhaité comporte des segments linéaires de trajet $S^{(i)}$, par exemple disposés bout à bout, et les associations prédéfinies associent des valeurs de référence $P_{ref}^{(i)}{}_{F/B}$ des paramètres de focalisation respectivement à chaque segment de trajet $S^{(i)}$.

A cet effet, les moyens de mise à jour 132 comportent tout d'abord une base de données 134 enregistrée dans la mémoire 116 et comportant les associations prédéfinies. En outre, les moyens de mise à jour 132 comportent des instructions, appelées instructions de sélection 136, pour fournir une valeur des paramètres de focalisation associée, par les associations prédéfinies, à au moins une position sur le trajet souhaité, à partir de la position $P_{B/O}$ du boîtier 110.

Plus précisément, dans le dispositif de sondage de la figure 1, les instructions de sélection 136 sont conçues pour, d'une part, sélectionner dans la base de données 134 un des segments de trajet souhaité $S^{(i)}$ à partir de la position $P_{B/O}$ du boîtier 110, et pour, d'autre part, fournir la valeur de référence $P_{ref}^{(i)}{}_{F/B}$ associée au segment de trajet $S^{(i)}$ sélectionné. A cet effet, les instructions de sélection 136 sont conçues pour, d'une part, déterminer si la position $P_{B/O}$ du boîtier 110 se trouve dans une sphère d'incertitude autour d'un des segments de trajet $S^{(i)}$, c'est-à-dire à proximité de ce segment de trajet $S^{(i)}$ selon une règle de proximité prédéfinie, et pour, d'autre part, sélectionner ce segment de trajet $S^{(i)}$ et fournir la valeur de référence $P_{ref}^{(i)}{}_{F/B}$ associée.

En outre, les moyens de mise à jour 132 sont conçus pour mettre à jour les paramètres de focalisation $P_{F/B}$ à partir de la valeur des paramètres de focalisation fournie par les moyens de sélection 136. Toujours dans le dispositif de sondage 100 de la figure 1, les moyens de mise à jour 132 sont ainsi conçus pour mettre à jour les paramètres de focalisation $P_{F/B}$ à la valeur de référence $P_{ref}^{(i)}{}_{F/B}$ fournie par les moyens de sélection 136.

Le programme 118 comporte en outre des instructions 138 de détermination d'une loi de retards R à partir, d'une part, des paramètres de focalisation $P_{F/B}$ fournis par les moyens de mise à jour 132 et, d'autre part, des positions des transducteurs par rapport au boîtier $P_{t/B}$.

Dans le dispositif de sondage 100 de la figure 1, les instructions 138 de détermination de la loi de retards comportent tout d'abord des instructions 140 pour déterminer les positions, notées $P_{t/O}$, des transducteurs par rapport à l'objet 102 à partir des positions $P_{t/B}$ des transducteurs par rapport au boîtier 110 et de la position $P_{B/O}$ du boîtier 110 par rapport à l'objet 102 : il s'agit d'un simple changement de repère. Les instructions 138 de détermination de la loi de retards R comportent en outre des instructions 142 pour déterminer des paramètres de focalisation, notés $P_{F/O}$, par rapport à l'objet 102 (les paramètres de focalisation $P_{F/O}$ représentant donc la position souhaitée du point focal F par rapport à l'objet 102) à partir des paramètres de focalisation $P_{F/B}$ et de la position $P_{B/O}$ du boîtier 110. Cette opération constitue donc également un simple changement de repère comme cela est connu en soi. Les instructions 138 de détermination de la loi de retards R comportent en outre des instructions 144 pour déterminer la loi de retards R à partir des paramètres de focalisation $P_{B/O}$ par rapport à l'objet 102 et des positions $P_{t/O}$ des transducteurs $120_1...120_N$ par rapport à l'objet 102. Cette opération comporte en particulier le calcul de trajets entre les transducteurs $120_1...120_N$ et la position souhaitée du point focal F, comme cela est connu en soi.

Enfin, le programme 118 comporte des instructions de commande 146 pour déterminer les signaux de commande C destinés aux transducteurs $120_1...120_N$, à partir de la loi de retards R. Plus précisément, les instructions 146 sont conçues de manière que les signaux de commande C présentent des retards les uns par rapport aux autres définis par la loi de retards R. Ainsi, grâce à ces retards dans les signaux de commande C, les ondes ultrasonores peuvent focaliser au point focal F positionné tel que souhaité.

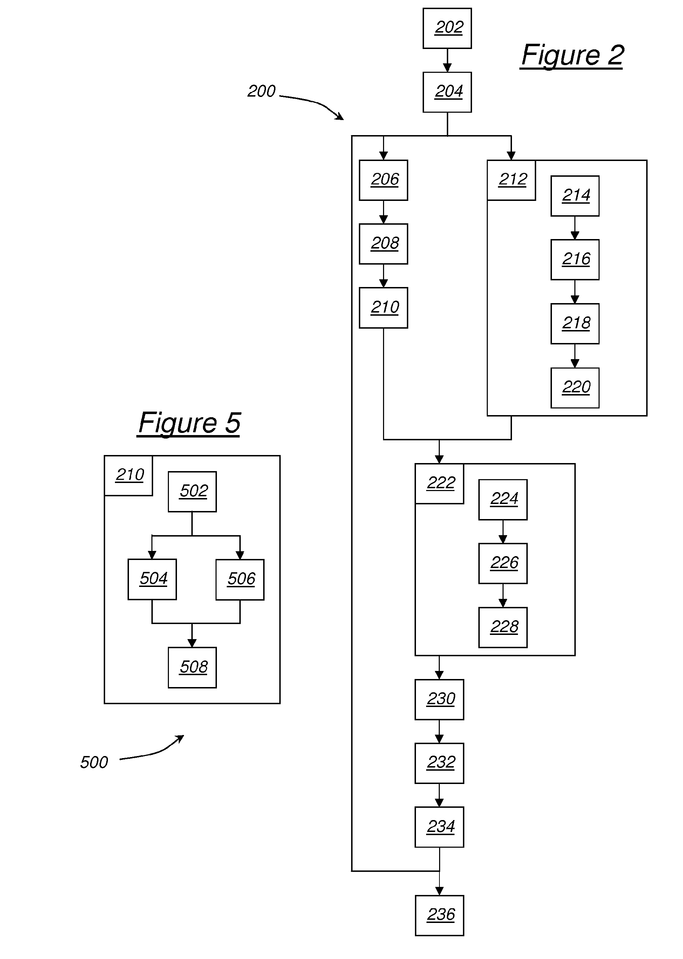

En référence à la figure 2, un procédé de sondage 200 de l'objet 102 mettant en oeuvre le dispositif de sondage 100 va à présent être décrit.

Au cours d'une étape 202, un opérateur configure les moyens de commande 108 du bras articulé 104 afin que ce dernier puisse déplacer la sonde 106 selon le trajet souhaité au contact de l'objet 102.

Au cours d'une étape 204, l'opérateur définit les associations entre des positions sur le trajet souhaité du boîtier 110 et des valeurs correspondantes des paramètres de focalisation. Dans l'exemple décrit, l'opérateur enregistre dans la base de données 134 les associations entre les segments de trajet $S^{(i)}$ du trajet souhaité et les valeurs de référence $P_{ref}^{(i)}{}_{F/B}$ des paramètres de focalisation.

On remarquera que les étapes 202 et 204 sont de préférences réalisées avant que le dispositif de sondage 100 ne débute le sondage proprement dit de l'objet 102. Par la suite, la sonde 106 se déplace par rapport à l'objet 102.

Au cours d'une étape 206, les moyens de localisation 130 déterminent la position $P_{B/O}$ du boîtier 110.

Au cours d'une étape 208, l'unité centrale de traitement 114 exécutant le programme de commande 118 reçoit la position $P_{B/O}$ du boîtier 110 de la part des moyens de localisation 130.

Au cours d'une étape 210, les moyens de mise à jour 132 mettent à jour les paramètres de focalisation $P_{F/B}$ à partir de la position $P_{B/O}$ du boîtier 110. Plus précisément, l'unité centrale de traitement 114 exécutant les instructions 136 fournit, à partir de la position $P_{B/O}$ du boîtier 110, une valeur des paramètres de focalisation associée, par les associations prédéfinies, à au moins une position sur le trajet souhaité. Les paramètres de focalisation $P_{F/B}$ sont mis à jour à cette valeur. Dans l'exemple décrit, l'unité centrale de traitement 114 exécutant les instructions de sélection 136 sélectionne dans la base de données 134 un des segments de trajet $S^{(i)}$ à partir de la position $P_{B/O}$ du boîtier 110, et fournit la valeur de référence $P_{ref}^{(i)}{}_{F/B}$ qui y est associée dans la base de données 134.

Au cours d'une étape 212, les moyens 122 de localisation des transducteurs $120_1...120_N$ déterminent les positions $P_{t/B}$ des transducteurs $120_1...120_N$. Plus précisément, cette étape 212 comporte les étapes 214 à 220 suivantes.

Au cours d'une étape 214, les émetteurs $124_1...124_N$ émettent des ondes de localisation.

Au cours d'une étape 216 les récepteurs 126 détectent les ondes de localisation.

Au cours d'une étape 218, l'unité centrale de traitement 114 exécutant le programme 118 reçoit les détections des récepteurs 126.

Au cours d'une étape 220, l'unité centrale de traitement 114 exécutant les instructions 128 détermine les positions $P_{t/B}$ des transducteurs $120_1...120_N$ à partir des détections des récepteurs 126.

Au cours d'une étape 222, l'unité centrale de traitement 114 exécutant les instructions 138 détermine la loi de retards R à partir, d'une part, des paramètres de focalisation $P_{F/B}$ mis à jour par les moyens de mise à jour 132 et, d'autre part, des positions $P_{t/B}$ des transducteurs $120_1...120_N$. Plus précisément, l'étape 222 comporte les étapes 224 à 228 suivantes.

Au cours d'une étape 224, l'unité centrale de traitement 114 exécutant les instructions 140 détermine les positions $P_{t/O}$ des transducteurs $120_1...120_N$ par rapport à l'objet 102 à partir des positions $P_{t/B}$ des transducteurs $120_1...120_N$ et de la position $P_{B/O}$ du boîtier 110.

Au cours d'une étape 226, l'unité centrale de traitement 114 exécutant les instructions 142 détermine des paramètres de focalisation $P_{F/O}$ par rapport à l'objet 102 à partir des paramètres de focalisation $P_{F/B}$ et de la position $P_{B/O}$ du boîtier 110.

Au cours d'une étape 228, l'unité centrale de traitement 114 exécutant les instructions 144 détermine la loi de retards R à partir des paramètres de focalisation $P_{F/O}$ par rapport à l'objet 102 et des positions $P_{t/O}$ des transducteurs $120_1...120_N$ par rapport à l'objet 102.

Au cours d'une étape 230, l'unité centrale de traitement 114 exécutant les instructions 146 provoque la fourniture aux transducteurs $120_1...120_N$, par le circuit électronique 112, de signaux de commande C présentant, les uns par rapport aux autres, des retards définis par la loi de retards R.

Au cours d'une étape 232, les transducteurs $120_1...120_N$, en réponse aux signaux de commande C, émettent des ondes ultrasonores dans l'objet 102, qui focalisent en un point focal F ayant la position souhaitée par rapport au boîtier 110, grâce aux retards des signaux de commande C entre eux.

Au cours d'une étape 234, les transducteurs $120_1...120_N$ reçoivent les échos des ondes ultrasonores qui ont été réfléchies sur et dans l'objet 102, et fournissent au programme de traitement 119 les signaux de mesure M correspondants.

Le procédé 200 retourne alors aux étapes 206 et 212.

En parallèle aux étapes 206 à 212, ou bien à leur suite, après que la sonde 106 a réalisé le trajet prévu, au cours d'une étape 236, l'unité centrale de traitement 114 exécutant les instructions du programme de traitement 119 traite les signaux de mesure M, par exemple dans le but de détecter des défauts de structure dans l'objet 102, en affichant les résultats du traitement sur un écran.

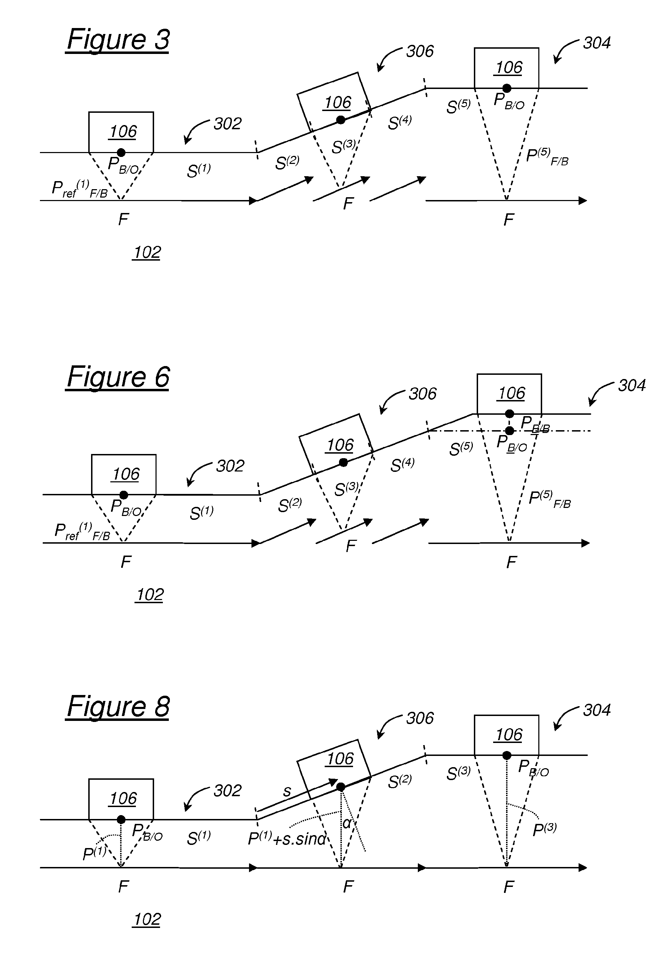

En référence à la figure 3, un exemple d'utilisation du dispositif 100 de la figure 1 va être décrit.

L'objet 102 comporte une face présentant, dans le plan de la figure 3, deux parties horizontales 302, 304 et une partie en biais 306 reliant les deux parties horizontales 302, 304.

Le trajet souhaité pour la sonde, correspondant à la forme connue de la face de l'objet 102, comporte cinq segments de trajets $S^{(1)}...S^{(5)}$, chacun associé à une valeur de référence $P^{(1)}_{F/B}...P^{(5)}_{F/B}$ respective de paramètres de focalisation. Ces associations sont enregistrées dans la base de données 134.

Le premier segment de trajet $S^{(1)}$ couvre la première partie horizontale 302, de sorte que le point focal F se déplace parallèlement à cette partie horizontale 302, à une profondeur de la partie horizontale 302 définie par la première valeur de référence $P^{(1)}_{F/B}$ associée au premier segment de trajet $S^{(1)}$.

Les trois segments de trajet suivants $S^{(2)}...S^{(4)}$ couvrent la partie en biais 306. Le point focal F se déplace parallèlement à la partie en biais 306 mais en étant ramené, au début de chaque segment de trajet $S^{(2)}...S^{(4)}$,

dans la continuité de son déplacement pour la première partie droite 302. Ceci est obtenu en choisissant des valeurs de référence $P^{(2)}_{F/B}...P^{(4)}_{F/B}$ définissant des positions du point focal F à des profondeurs croissantes et avec un angle adapté à la partie en biais 306.

Le cinquième segment de trajet $S^{(5)}$ couvre la seconde partie droite 304. Le point focal F se déplace parallèlement à cette partie horizontale 304, à une profondeur de la partie horizontale 304 définie par la cinquième valeur de référence $P^{(5)}_{F/B}$, de manière que le point focal F se déplace dans la continuité de son déplacement pour la première partie horizontale 302.

Ainsi, le point focal F se déplace relativement linéairement lorsque la sonde 106 se déplace sur la face de l'objet 102, malgré le fait que la surface de l'objet 102 n'est pas une ligne uniquement horizontale. Selon ce mode de réalisation, on comprend que l'on améliore la linéarité du déplacement du point focal F dans l'objet 102 en augmentant le nombre de segments associés à des paramètres de focalisation dans les parties du trajet souhaité qui ne sont pas parallèles au déplacement souhaité du point focal F. En particulier, dans l'exemple de la figure 3, plus le nombre de segments couvrant la partie en biais 306 est important (ici trois), plus le trajet effectivement suivi par le point focal F dans l'objet 102 est proche du trajet rectiligne horizontal idéalement souhaité.

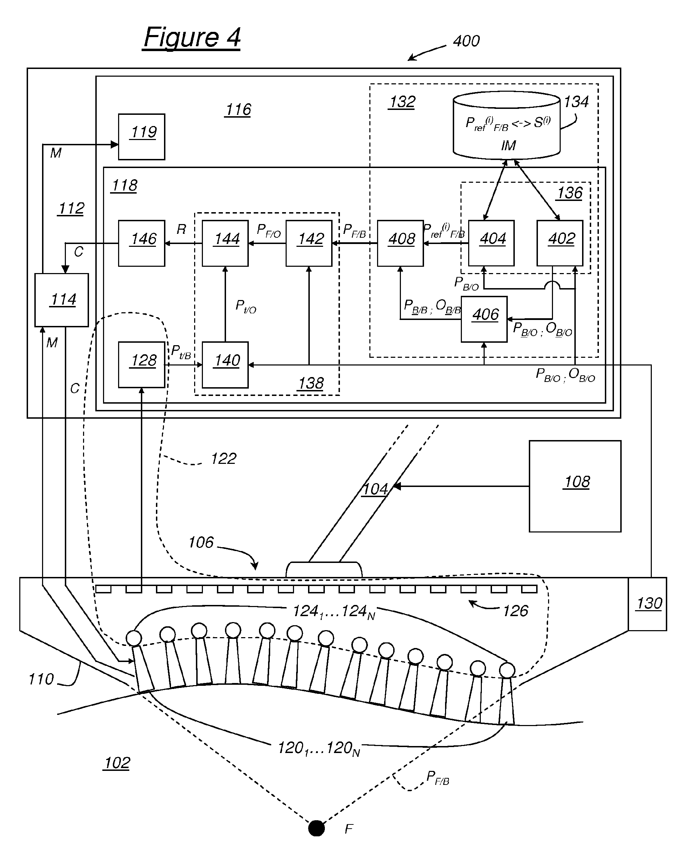

En référence à la figure 4, un dispositif de sondage 400 selon un deuxième mode de réalisation de l'invention comporte les mêmes éléments que le dispositif de la figure 1, pour lesquels les mêmes références sont utilisées, sauf pour les éléments qui vont à présent être décrits. Selon ce deuxième mode de réalisation, le dispositif de sondage 400 est conçu pour prendre en compte un écart entre une position et/ou une orientation réelle du boîtier 110, d'une part, et une position et/ou une orientation souhaitée sur le trajet souhaité, d'autre part, dans la mise à jour des paramètres de focalisation $P_{F/B}$.

Ainsi, dans le dispositif de sondage 400 de la figure 4, les moyens de localisation 130 sont non seulement conçus pour fournir une position $P_{B/O}$ du boîtier par rapport à l'objet 102, mais en outre conçus pour fournir une orientation $O_{B/O}$ du boîtier 110 par rapport à l'objet 102.

Par ailleurs, les moyens de mise à jour 132 comportent toujours la base de données 134, mais cette dernière comporte en outre à présent des informations de mouvement souhaité, notées IM, définissant un mouvement souhaité du boîtier 110 par rapport à l'objet 102. Les informations de mouvement souhaité IM comportent en particulier des informations de trajet souhaité définissant le trajet souhaité du boîtier 110 par rapport à l'objet 102, et des informations d'orientation souhaitée définissant l'orientation souhaitée du boîtier 110 par rapport à l'objet 102 le long du trajet souhaité. Dans le dispositif de la figure 4, les informations de trajet souhaité comportent les segments de trajet $S^{(i)}$ des associations prédéfinies.

Les instructions de sélection 136 comportent en outre des instructions 402, appelée instructions de recalage 402, pour déterminer une position recalée du boîtier 110 par rapport à l'objet 102 sur le trajet souhaité, cette position recalée étant notée $P_{B/O}$. La position recalée $P_{B/O}$ est déterminée à partir de la position $P_{B/O}$ du boîtier 110, fournie par les moyens de localisation 130, et des informations de trajet souhaité de la base de données 134. Par exemple, la position recalée est le point du trajet souhaité le plus proche de la position du boîtier par rapport à l'objet $P_{B/O}$. Les instructions de recalage 402 sont en outre conçues pour déterminer une orientation recalée, notée $O_{B/O}$, du boîtier 110 sur l'orientation souhaitée du boîtier 110, à partir de l'orientation $O_{B/O}$ du boîtier 110 et des informations d'orientation souhaitée de la base de données 134.

Dans le dispositif de sondage 400 de la figure 4, les instructions de sélection 136 comportent en outre des instructions, appelées instructions de sélection 404, pour sélectionner un des segments de trajet $S^{(i)}$ à partir de la position non recalée $P_{B/O}$ et des informations de trajet souhaité de la base de données 134. Par ailleurs, les instructions de sélection 136 sont conçues pour fournir la valeur de référence $P_{ref}^{(i)}{}_{F/B}$ associée au segment de trajet $S^{(i)}$ sélectionné.

Les moyens de mise à jour 132 comportent en outre des instructions du programme de commande 118, appelées instructions de détermination d'écart 406, pour déterminer l'écart de position $P_{B/B}$ entre la position $P_{B/O}$ du boîtier 110 et la position recalée $P_{B/O}$ du boîtier 110, ainsi que l'écart d'orientation, noté $O_{B/B}$, entre l'orientation $O_{B/O}$ du boîtier 110 et son orientation recalée $O_{B/O}$.

Les moyens de mise à jour 132 comportent en outre des instructions du programme de commande 118, appelées instructions de correction 408, pour corriger la valeur des paramètres de focalisation fournie par les instructions de sélection 136 à partir de l'écart de position $P_{B/B}$ et de l'écart d'orientation $O_{B/B}$. Dans l'exemple décrit, la valeur des paramètres de focalisation fournie par les instructions de sélection 136 est, comme expliqué précédemment, l'une des valeurs de référence $P_{ref}^{(i)}{}_{F/B}$ de la base de données 134.

Les moyens de mise à jour 132 sont ainsi conçus pour mettre à jour les paramètres de focalisation $P_{F/B}$ à la valeur des paramètres de focalisation corrigée par les instructions de correction 408.

Les paramètres de focalisation $P_{F/B}$ ainsi mis à jour permettent de positionner le point focal F au même endroit que si le boîtier 110 avait sa position souhaitée et son orientation souhaitée.

En référence à la figure 5, un procédé de sondage 500 de l'objet 102 mettant en oeuvre le dispositif de sondage 400 comporte les mêmes étapes que le procédé 200 de la figure 2, sauf pour l'étape 210 qui comporte à présent les étapes suivantes pour mettre à jour les paramètres de focalisation $P_{F/B}$ en fonction de la position $P_{B/O}$ du boîtier 110 et de son orientation $O_{B/O}$.

Au cours d'une étape 502, l'unité centrale de traitement 114 exécutant les instructions 402 détermine la position recalée $P_{B/O}$ et l'orientation recalée $O_{B/O}$ du boîtier 110, à partir des informations de mouvement souhaité IM de la base de données 134, de la position $P_{B/O}$ du boîtier 110 et de son orientation $O_{B/O}$.

Au cours d'une étape 504, l'unité centrale de traitement 114 exécutant les instructions 404 sélectionne, dans la base de données 134, un segment de trajet $S^{(i)}$ à partir de la position non recalée $P_{B/O}$ du boîtier 110, et fournit la valeur de référence $P_{ref}^{(i)}{}_{F/B}$ des paramètres de focalisation associée à ce segment de trajet $S^{(i)}$.

Parallèlement à l'étape 504, au cours d'une étape 506, l'unité centrale de traitement 114 exécutant les instructions 406 détermine l'écart de position $P_{B/B}$ et l'écart d'orientation $O_{B/B}$.

Au cours d'une étape 506, l'unité centrale de traitement 114 exécutant les instructions 408 corrige la valeur de référence des paramètres de focalisation à partir des écarts de position $P_{B/B}$ et d'orientation $O_{B/B}$. Cette valeur corrigée constitue alors les paramètres de focalisation $P_{F/B}$ mis à jour.

En référence à la figure 6, un exemple similaire à celui de la figure 3 va être décrit, afin d'illustrer la correction d'une erreur de positionnement. L'homme du métier pourra aisément adapter cet exemple à une correction d'orientation.

La sonde 106 est destinée à suivre le trajet souhaité constitué des cinq segments de trajet $S^{(1)}...S^{(5)}$ (le segment de trajet $S^{(5)}$ étant représenté en trait interrompu mixte sur cette figure). Sur la partie 304 de l'objet 102, la sonde 106 a dévié du trajet souhaité d'un écart représenté par $P_{B/B}$ compte tenu du fait que la face réelle de l'objet 102 sur laquelle elle se déplace ne correspond pas exactement au trajet souhaité enregistré dans la base de données 134.

Cet écart peut provenir du fait que les pièces admettent des tolérances d'usinage qui peuvent être supérieures aux précisions nécessaires pour le sondage ou d'une erreur de positionnement de la pièce. En outre, la sonde 106 est susceptible de ne pas suivre exactement la surface souhaitée. En l'absence de correction, ces écarts se traduiraient par une mauvaise focalisation du faisceau d'ondes ultrasonores dans la zone souhaitée car les retards calculés ne seraient pas adaptés aux points focaux souhaités.

Dans la présente invention, grâce à la correction d'écart prévue dans les instructions 138, le point focal F n'est cependant pas affecté par l'erreur de positionnement de la sonde 106. Cette solution de correction se substitue avantageusement à une correction mécanique, sur le bras articulé 104 par exemple.

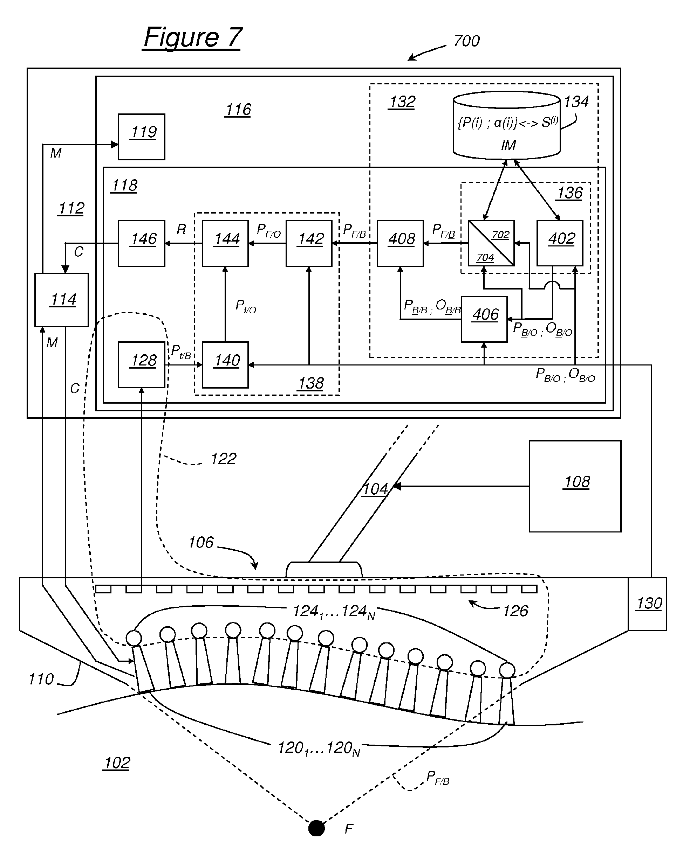

En référence à la figure 7, un dispositif de sondage 700 selon un troisième mode de réalisation de l'invention comporte les mêmes éléments que le dispositif de la figure 4, pour lesquels les mêmes références sont utilisées, sauf pour les éléments qui vont à présent être décrits. Comme dans le deuxième mode de réalisation, le dispositif de sondage 700 est conçu pour prendre en compte un écart entre une position et/ou une orientation réelle du boîtier 110, d'une part, et une position et/ou une orientation souhaitée sur le trajet souhaité, d'autre part, dans la mise à jour des paramètres de focalisation $P_{F/B}$. En revanche, contrairement aux premier et deuxième modes de réalisation, les segments de trajet souhaité $S^{(i)}$ ne sont pas associés directement à des paramètres de focalisation $P_{ref}^{(i)}{}_{F/B}$ déjà calculés mais à des paramètres d'une opération de recalage donnant, par le calcul, une valeur des paramètres de focalisation à partir de chaque position sur le trajet souhaité. Selon ce mode de réalisation, on comprend que l'on améliore la précision du déplacement du point focal F dans l'objet 102 sans avoir besoin d'augmenter le nombre de segments $S^{(i)}$ dans les parties du trajet souhaité qui ne sont pas parallèles au déplacement souhaité du point focal F. En particulier, dans l'exemple de la figure 8 qui illustre le trajet bidimensionnel 302, 306, 304 de la sonde du dispositif 700, un seul segment peut désormais couvrir la partie en biais 306 puisque la correspondance (i.e. le changement de repère) entre la partie 306 et la trajectoire idéalement suivie par le point focal F peut être complètement déterminée à l'aide d'une opération de recalage définie par une application affine.

Dans la suite de la description, pour simplifier les notations, seules les coordonnées bidimensionnelles dans le plan des figures seront utilisés, l'homme du métier pouvant aisément adapter l'enseignement qui suit aux trois dimensions. Ainsi, les paramètres de focalisation $P_{F/B}$, c'est-à-dire les coordonnées successives du point de focalisation F par rapport au boîtier 110, peuvent chacun être représentés par une profondeur P par rapport au boîtier 110 et un angle $\alpha$ représentant l'orientation du boîtier 110 par rapport à une direction prédéterminée, par exemple la verticale. Comme dans les exemples précédents, le trajet souhaité comporte des segments linéaires de trajet $S^{(i)}$, par exemple disposés bout à bout. Dans la base de données 134, des informations de focalisation sont associées à chaque segment. Dans le dispositif de sondage 700, les informations de focalisation associées à un segment de trajet $S^{(i)}$ comportent des paramètres d'une opération de recalage permettant de calculer une valeur des paramètres de focalisation à partir de chaque position sur ce segment de trajet $S^{(i)}$, notamment des paramètres d'une application affine tels qu'une profondeur P(i) et un angle $\alpha(i)$. L'angle $\alpha(i)$ représente la pente du segment et permet, d'une part, de définir la direction dans laquelle se trouve le point focal F par rapport au boîtier 110 et, d'autre part, de corriger la profondeur P(i) lorsque le boîtier se déplace le long du segment $S^{(i)}$, comme cela sera expliqué par la suite.

Ainsi, dans le dispositif de sondage 700 de la figure 7, les instructions de sélection 404 sont remplacées par des instructions 702 et 704.

Les instructions 702 sont conçues pour sélectionner un des segments de trajet $S^{(i)}$ enregistrés dans la base de données à partir de la position non recalée $P_{B/O}$. Par ailleurs, les instructions 702 sont conçues pour fournir les informations de focalisation associées au segment de trajet $S^{(i)}$ sélectionné, c'est-à-dire, dans l'exemple décrit, la profondeur P(i) et l'angle $\alpha(i)$ associés au segment de trajet $S^{(i)}$ sélectionné.

Les instructions 704 sont conçues pour calculer, au moyen d'une formule mathématique de recalage, en l'occurrence une application mathématique affine définie par P(i) et $\alpha(i)$, la position $P_{F/B}$ du point focal F à partir de la position recalée $P_{B/O}$ du boîtier 110 sur le segment de trajet $S^{(i)}$ sélectionné. Dans le dispositif de sondage 700, la formule donnant la position $P_{F/B}$ du point focal F correspondant à une position s sur le trajet $S^{(i)}$ est : $P_{F/B} = P(i) + s.\sin \alpha(i)$.

Ainsi, dans le dispositif de sondage 700, les associations prédéfinies comportent l'ensemble constitué, d'une part, des informations de focalisation enregistrées dans la base de données 134 (P(i) et $\alpha(i)$) et, d'autre part, d'une application mathématique elle-même définie par ces informations de focalisation, ces deux éléments associant des paramètres de focalisation à chaque position du boîtier sur son trajet.

En référence à la figure 8, un exemple d'utilisation du dispositif 700 de la figure 7 va être décrit.

L'objet 102 comporte une face présentant, dans le plan de la figure 7, deux parties horizontales 302, 304 et une partie en biais 306 reliant les deux parties horizontales 302, 304. Le point focal F doit, lui, suivre une trajectoire rectiligne à profondeur constante dans l'objet 102, soit à une distance $P_A$ de la partie horizontale 302 et à une distance $P_B$ de la partie horizontale 304.

Le trajet souhaité comporte trois segments de trajets $S^{(1)}...S^{(3)}$, définis pour couvrir chacun respectivement les parties 302, 306 et 304, chacun associé à des informations de focalisation $\{P(1) ; \alpha(1)\} ... \{P(3) ; \alpha(3)\}$ respectives valant :

$$\{P(1) ; \alpha(1)\} = \{P_A ; 0\},$$

$$\{P(2) ; \alpha(2)\} = \{P_A ; \alpha\},$$

et

$$\{P(3) ; \alpha(3)\} = \{P_B ; 0\}.$$

Concrètement, les paramètres P(i), $\alpha(i)$ permettent de réaliser le changement de repère entre le trajet souhaité défini par les trois segments $S^{(1)}...S^{(3)}$ et la trajectoire rectiligne horizontale qui doit être suivie par le point focal F.

Ainsi, le premier segment de trajet $S^{(1)}$ couvre la première partie horizontale 302, parallèle et située à la distance $P_A$ de la trajectoire de focalisation. Les instructions 704 calculent la position du point focal par rapport au boîtier. Comme l'angle $\alpha(1)$ est nul, cela signifie qu'il n'y a pas de correction à apporter à la profondeur

$P(1) = P_A$ et donc que le point focal reste à profondeur constante $P_A$ et se déplace parallèlement à la première partie horizontale 302.

Le segment de trajet suivant $S^{(2)}$ couvre la partie en biais 306. Les instructions 704 calculent la position du point focal par rapport au boîtier. Comme l'angle $\alpha(2) = \alpha$ n'est pas nul, à chaque position s du boîtier 110 sur le segment $S^{(2)}$ les instructions 704 fournissent la position calculée au moyen de la formule $P_{F/B} = P(2) + s.\sin \alpha(2) = P_A + s.\sin \alpha$ afin de corriger la pente de la partie 306. Ainsi, le point focal F se déplace linéairement et horizontalement dans la continuité de son déplacement parallèlement au premier segment $S^{(1)}$.

Le troisième segment de trajet $S^{(3)}$ couvre la partie horizontale 304, parallèle et située à la distance $P_B$ de la trajectoire de focalisation. Les instructions 704 calculent la position du point focal par rapport au boîtier. Comme l'angle $\alpha(3)$ est nul, cela signifie qu'il n'y a pas de correction à apporter à la profondeur $P(3) = P_B$ et donc que le point focal F reste à profondeur constante et se déplace parallèlement à la partie horizontale 304 à la distance $P_B$ de cette dernière dans la continuité de ses déplacements précédents.

Ainsi, grâce aux informations de focalisation $\{P(1) ; \alpha(1)\} ... \{P(3) ; \alpha(3)\}$ qui comportent en fait des paramètres de changement de repères associés aux segments de trajet prédéfinis $S^{(1)}...S^{(3)}$, le point focal F se déplace linéairement et horizontalement lorsque la sonde 106 se déplace sur la surface de l'objet 102, malgré le fait que la surface de l'objet 102 n'est pas une ligne uniquement horizontale.

On note que dans l'exemple de la figure 8, la sonde 106 se déplace effectivement sur le trajet souhaité tel que prévu dans la base de données 134. Mais comme dans le deuxième mode de réalisation et comme cela a été précisé précédemment, le dispositif de sondage 700 est conçu pour prendre en compte un écart entre une position et/ou une orientation réelle du boîtier 110, d'une part, et une position et/ou une orientation souhaitée sur le trajet souhaité, d'autre part, dans la mise à jour des paramètres de focalisation $P_{F/B}$. Ainsi, si la sonde 106 déviait comme dans la partie 304 de la figure 6 par rapport au trajet souhaité, cet écart pourrait être mesuré et calculé à l'aide des moyens de localisation 130 et des instructions de détermination d'écart 406, pour être ensuite pris en compte dans la mise à jour des paramètres de focalisation $P_{F/B}$ par les instructions de correction 408.

Par ailleurs et avantageusement, les paramètres de correction utilisés par les instructions de correction 408 peuvent être de même nature que les informations de focalisation enregistrées dans la base de données 134 et exploitées par les instructions 702 et 704. Dans l'exemple précédemment détaillé du troisième mode de réalisation, les informations de focalisation sont des paramètres de distance $P(i)$ et d'angle $\alpha(i)$ qui sont effectivement de même nature respectivement que les paramètres de correction $P_{B/B}$ (écart de position) et $O_{B/B}$ (écart d'orientation) fournis par les instructions de détermination d'écart 406 aux instructions de correction 408. Ainsi, la correction apportée par les instructions de correction 408 peut revenir à simplement corriger les informations de focalisation $P(i)$ et $\alpha(i)$ enregistrées dans la base de données 134 à l'aide des valeurs $P_{B/B}$ et $O_{B/B}$ mesurées en utilisant la même formule de recalage (application affine) que celle décrite précédemment.

La contribution respective des informations de focalisation (pré-enregistrées dans la base de données 134) et des paramètres de correction (mesurés à l'aide des moyens de localisation 130) peut même dans ce troisième mode de réalisation être modulée à volonté, la correction réalisée par les instructions 408 pouvant compenser des imprécisions ou insuffisances sur les données pré-enregistrées. Il n'est ainsi pas nécessaire d'avoir une connaissance a priori très précise de la forme de l'objet 102 puisque la correction d'écart prévue dans les instructions 408 se fait automatiquement sans nécessiter de recours à une correction mécanique.

A l'extrême, la forme réelle non plane de l'objet 102 pourrait même ne pas être prise en compte du tout dans la base de données 134, le trajet souhaité étant alors enregistré comme rectiligne et horizontal et toujours parallèle à la trajectoire que doit suivre le point de focalisation F dans l'objet 102. Dans ce cas, en prenant comme référence la partie horizontale 302, le déplacement de la sonde 106 sur les parties 306 et 304 serait considéré par le dispositif de sondage 700 comme un écart par rapport au trajet souhaité (qui est alors une droite horizontale prolongeant la partie 302) et serait complètement traité comme tel par les instructions de correction 408 pour lesquelles les valeurs $P_{B/B}$ et $O_{B/B}$ contribueraient complètement à l'opération de recalage définie par la formule mathématique affine de mise à jour des paramètres de focalisation.

Il apparaît clairement qu'un dispositif de sondage à ultrasons tel que ceux décrits précédemment permet de prévoir une trajectoire du point focal indépendante de la surface de l'objet, et ainsi effectuer des contrôles de l'objet complexes.

On notera par ailleurs que l'invention n'est pas limitée aux modes de réalisation décrits précédemment.

En particulier, les instructions de programme d'ordinateur pourraient être remplacées par des circuits électroniques conçus pour réaliser les mêmes fonctions.

En outre, les positions et orientations décrites précédemment pourraient être exprimées de manière indirecte en passant par un référentiel extérieur au système par exemple lié à un bâti supposé fixe. Par exemple, la position $P_{B/O}$ du boîtier 110 par rapport à l'objet 102 pourrait être exprimée par les deux positions suivantes : la position du boîtier 110 par rapport au référentiel extérieur et la position de l'objet 102 par rapport au référentiel extérieur.

En outre, des segments de trajet non linéaires pourraient être utilisés.

En outre, les paramètres de focalisation peuvent être exprimés par rapport au boîtier comme décrit précédemment, ou bien par rapport à l'objet à sonder. Dans ce dernier cas, la position du boîtier par rapport à l'objet est utilisée pour retrouver la position du point focal par rapport au boîtier.

En outre, plutôt que des valeurs discrètes de paramètres de focalisation associées à des segments de trajets, les associations prédéfinies pourraient comporter une fonction continue donnant la valeur des paramètres de focalisation en fonction de la position de boîtier. Par exemple, dans le cas de la figure 3, cette fonction pourrait être une fonction affine définissant des positions du point focal F à des profondeurs croissantes dans le repère du boîtier et constantes dans le repère de l'objet, en présentant un angle adapté à la partie en biais. Ainsi, le déplacement du point focal resterait horizontal sur les trois parties de l'objet, et en particulier le long de la partie en biais.

Enfin, il apparaîtra en effet à l'homme de l'art que diverses modifications peuvent être apportées aux modes de réalisation décrits ci-dessus, à la lumière de l'enseignement qui vient de lui être divulgué. Dans les revendications qui suivent, les termes utilisés ne doivent pas être interprétés comme limitant les revendications aux modes de réalisation exposés dans la présente description, mais doivent être interprétés pour y inclure tous les équivalents que les revendications visent à couvrir du fait de leur formulation et dont la prévision est à la portée de l'homme de l'art en appliquant ses connaissances générales à la mise en oeuvre de l'enseignement qui vient de lui être divulgué.

## Revendications

**1.** Dispositif de sondage à ultrasons comportant :

- un boîtier (110),
- des moyens de localisation (130) conçus pour fournir une position $P_{B/O}$ du boîtier (110) par rapport à un référentiel lié à un objet à sonder (102),
- des moyens (114, 138) de détermination d'une loi de retards (R) à partir d'une position souhaitée d'un point focal (F) par rapport à un référentiel lié au boîtier (110), cette position souhaitée étant représentée par des paramètres de focalisation notés $P_{F/B}$,
- des moyens de commande (114, 146) conçus pour fournir des signaux de commande (C) à partir de la loi de retards (R),
- des transducteurs ($120_1$...$120_N$) attachés au boîtier (110), conçus pour recevoir les signaux de commande et pour, en réponse, émettre dans l'objet (102) des ondes ultrasonores retardées les unes par rapport aux autres suivant la loi de retards de manière qu'elles focalisent au point focal (F) défini par les paramètres de focalisation $P_{F/B}$,

**caractérisé en ce que** :

- il comporte des moyens (134) de stockage :

• de positions prédéterminées ($S^{(i)}$) sur un trajet souhaité du boîtier (110) par rapport au référentiel lié à l'objet (102), et
• d'associations prédéfinies de plusieurs positions différentes souhaitées $P_{ref}^{(i)}{}_{F/B}$) du point focal (F) par rapport au référentiel lié au boîtier (110) à plusieurs positions différentes parmi lesdites positions prédéterminées ($S^{(i)}$) sur ce trajet souhaité,

- il comporte en outre des moyens (132) de mise à jour de la position souhaitée ($P_{ref}^{(i)}{}_{F/B}$) du point focal (F) par rapport au référentiel lié au boîtier et donc des paramètres de focalisation $P_{F/B}$ correspondants à partir, d'une part, desdites associations prédéfinies stockées, et, d'autre part, de la position $P_{B/O}$ du boîtier (110), et
- les moyens de détermination (114, 138) sont conçus pour une mise à jour de la loi de retards (R) à partir de la mise à jour de la position souhaitée du point focal (F) par rapport au référentiel lié au boîtier (110) et des paramètres de focalisation $P_{F/B}$ correspondants.

**2.** Dispositif de sondage à ultrasons selon la revendication 1, dans lequel les moyens de localisation (130) du boîtier (110) comportent un capteur conçu pour mesurer la position $P_{B/O}$ du boîtier (110).

**3.** Dispositif de sondage à ultrasons selon la revendication 1 ou 2, dans lequel les moyens de mise à jour (132) comportent des moyens de sélection (114, 136) conçus pour fournir une valeur ($P_{ref}^{(i)}{}_{F/B}$) des paramètres de focalisation associée, par les associations prédéfinies, à au moins une position ($S^{(i)}$) sur le trajet souhaité, à partir de la position $P_{B/O}$ du boîtier (110).

**4.** Dispositif de sondage à ultrasons selon la revendication 3, dans lequel les moyens de mise à jour (132) sont conçus pour mettre à jour les paramètres de focalisation $P_{F/B}$ à la valeur ($P_{ref}^{(i)}{}_{F/B}$) des paramètres de focalisation fournie par les moyens de sélection (114, 136).

**5.** Dispositif de sondage à ultrasons selon la revendication 3, dans lequel les moyens de mise à jour (132) comportent en outre :

- des moyens de recalage (114, 402) conçus pour déterminer une position du boîtier (110) par rapport au référentiel lié à l'objet (102) recalée sur le trajet souhaité à partir de la position $P_{B/O}$ du boîtier (110), cette position recalée étant notée $P_{B/O}$, et/ou une orientation du boîtier (110) recalée sur une orientation souhaitée du boîtier (110) à partir de l'orientation $O_{B/O}$ du boîtier (110), cette orientation recalée étant notée $\overline{O}_{B/O}$,

- des moyens de détermination d'écart (114, 406) conçus pour déterminer un écart de position, noté $P_{B/B}$, entre la position $P_{B/O}$ du boîtier (110) et la position recalée $\overline{P}_{B/O}$, et/ou un écart d'orientation, noté $O_{B/B}$, entre l'orientation $O_{B/O}$ du boîtier (110) et l'orientation recalée $\overline{O}_{B/O}$,

- des moyens de correction (114, 408) conçus pour corriger la valeur $(P_{ref}^{(i)}{}_{F/B})$ des paramètres de focalisation fournie par les moyens de sélection (136) à partir de l'écart de position $P_{B/B}$ et/ou de l'écart d'orientation $O_{B/B}$,

et dans lequel les moyens de mise à jour (132) sont conçus pour mettre à jour les paramètres de focalisation $P_{F/B}$ à la valeur corrigée des paramètres de focalisation fournie par les moyens de correction (114, 408).

6. Dispositif de sondage à ultrasons selon l'une des revendications 3 à 5, dans lequel :

- le trajet souhaité comportant des segments de trajet $S^{(i)}$, les associations prédéfinies associent des valeurs de référence $P_{ref}^{(i)}{}_{F/B}$ des paramètres de focalisation et donc d'une position souhaitée du point focal par rapport au référentiel lié au boîtier respectivement à chaque segment de trajet $S^{(i)}$,

- les moyens de sélection (114, 136) sont conçus pour sélectionner un des segments de trajet $S^{(i)}$ à partir de la position $P_{B/O}$ du boîtier (110) et pour fournir la valeur de référence $P_{ref}^{(i)}{}_{F/B}$ associée au segment de trajet $S^{(i)}$ sélectionné.

7. Dispositif de sondage à ultrasons selon l'une quelconque des revendications 3 à 6, dans lequel les associations prédéfinies comportent des paramètres d'une opération de recalage donnant une valeur des paramètres de focalisation à partir d'une position sur le trajet souhaité et dans lequel les moyens de sélections (114, 136) comportent des moyens (114, 704) conçus pour calculer, au moyen des paramètres de l'opération de recalage, la valeur des paramètres de focalisation à partir d'une position sur le trajet souhaité déterminée à partir de la position $P_{B/O}$ du boîtier (110).

8. Dispositif de sondage à ultrasons selon l'une des revendications 1 à 7, dans lequel les transducteurs $(120_1...120_N)$ sont attachés de manière mobile au boîtier (110), comportant des moyens (122) de localisation des transducteurs $(120_1...120_N)$ conçus pour déterminer des positions $P_{t/B}$ des transducteurs

$(120_1...120_N)$ par rapport à un référentiel lié au boîtier (110), et dans lequel les moyens (114, 138) de détermination de la loi de retards sont conçus pour déterminer la loi de retards à partir en outre de ces positions $P_{t/B}$ des transducteurs $(120_1...120_N)$.

9. Procédé de commande de transducteurs $(120_1...120_N)$ d'une sonde à ultrasons, comportant les étapes suivantes :

- recevoir (208) une position $P_{B/O}$ d'un boîtier (110) de la sonde par rapport à un référentiel lié à un objet à sonder (102), les transducteurs $(120_1...120_N)$ étant attachés à ce boîtier (110),

- déterminer (222) une loi de retards (R) à partir d'une position souhaitée d'un point focal (F) par rapport à un référentiel lié au boîtier (110), cette position souhaitée étant représentée par des paramètres de focalisation notés $P_{F/B}$,

- fournir (230) des signaux de commande (C) aux transducteurs $(120_1... 120_N)$ à partir de la loi de retards,

**caractérisé en ce qu'**il comporte en outre :

- une étape (204) de définition et de stockage :

• de positions prédéterminées $(S^{(i)}$ sur un trajet souhaité du boîtier (110) par rapport au référentiel lié à l'objet (102), et

• d'associations prédéfinies de plusieurs positions différentes souhaitées $P_{ref}^{(i)}{}_{F/B})$ du point focal (F) par rapport au référentiel lié au boîtier (110) à plusieurs positions différentes parmi lesdites positions prédéterminées $(S^{(i)})$ sur ce trajet souhaité,

- une étape (210) de mise à jour de la position souhaitée $(P_{ref}^{(i)}{}_{F/B})$ du point focal (F) par rapport au référentiel lié au boîtier et donc des paramètres de focalisation $P_{F/B}$ correspondants, à partir, d'une part, desdites associations prédéfinies stockées et, d'autre part, de la position $P_{B/O}$ du boîtier (110), et

- une étape (222) de mise à jour de la loi de retards (R) à partir de la mise à jour de la position souhaitée du point focal (F) par rapport au référentiel lié au boîtier (110) et des paramètres de focalisation $P_{F/B}$ correspondants.

10. Programme d'ordinateur téléchargeable depuis un réseau de communication et/ou enregistré sur un support lisible par ordinateur et/ou exécutable par un processeur, **caractérisé en ce qu'**il comprend des instructions pour la mise en oeuvre d'un procédé de commande de transducteurs d'une sonde à ultrasons selon la revendication 9, lorsque ledit programme est exécuté par un ordinateur.

**Patentansprüche**

1. Ultraschallsondiervorrichtung, die Folgendes umfasst:

    - ein Gehäuse (110),
    - Ortungsmittel (130), die konzipiert sind, um eine Position $P_{B/O}$ des Gehäuses (110) in Bezug zu einem Referenzwerk, das mit einem zu sondierenden Objekt (102) verbunden ist, zu liefern,
    - Mittel (114, 138) zum Bestimmen eines Gesetzes der Verzögerungen (R) ausgehend von einer gewünschten Position eines Brennpunkts (F) in Bezug zu einem Referenzwerk, das mit dem Gehäuse (110) verbunden ist, wobei diese gewünschte Position durch Fokussierungsparameter mit der Bezeichnung $P_{F/B}$ dargestellt ist,
    - Steuermittel (114, 146), die konzipiert sind, um Steuersignale (C) ausgehend von dem Gesetz der Verzögerungen (R) zu liefern,
    - Messgrößenumformer ($120_1$ ... $120_N$), die an dem Gehäuse (110) angebracht sind, die konzipiert sind, um Steuersignale zu empfangen und um, als Reaktion, in das Objekt (102) Ultraschallwellen, die voneinander gemäß dem Gesetz der Verzögerungen verzögert sind, derart zu senden, dass sie sich im Brennpunkt (F), der durch die Fokussierungsparameter $P_{F/B}$ definiert sind, fokussieren, **dadurch gekennzeichnet, dass**:
    - sie Mittel (134) zum Speichern umfasst:

        -- für vorbestimmte Positionen ($S^{(i)}$) auf dem gewünschten Verlauf des Gehäuses (110) in Bezug zu dem Referenzwerk, das mit dem Objekt (102) verbunden ist, und
        -- vordefinierter Assoziationen mehrerer unterschiedlicher gewünschter Positionen $P_{ref}^{(i)}{}_{F/B}$ des Brennpunkts (F) in Bezug zu dem Referenzwerk, das mit dem Gehäuse (110) verbunden ist, mit mehreren unterschiedlichen Positionen unter den vorbestimmten Positionen ($S^{(i)}$) auf dem gewünschten Verlauf,

        - sie außerdem Mittel (132) zum Aktualisieren der gewünschten Position ($P_{ref}^{(i)}{}_{F/B}$) des Brennpunkts (F) in Bezug zu dem Referenzwerk, das mit dem Gehäuse verbunden ist, umfasst, und daher entsprechende Fokussierungsparameter $P_{F/B}$, ausgehend einerseits von den gespeicherten vordefinierten Assoziationen und, andererseits, der Position $P_{B/O}$ des Gehäuses (110), und
        - wobei die Mittel zum Bestimmen (114, 138) für eine Aktualisierung des Gesetzes der Verzögerungen (R) ausgehend von der Aktualisierung der gewünschten Position des Brennpunkts (F) in Bezug zu dem Referenzwerk, das mit dem Gehäuse (110) verbunden ist, und entsprechenden Fokussierungsparametern $P_{F/B}$ konzipiert sind.

2. Ultraschallsondiervorrichtung nach Anspruch 1, wobei die Ortungsmittel (130) des Gehäuses (110) einen Sensor umfassen, der konzipiert ist, um die Position $P_{B/O}$ des Gehäuses (110) zu erfassen.

3. Ultraschallsondiervorrichtung nach Anspruch 1 oder 2, wobei die Aktualisierungsmittel (132) Auswahlmittel (114, 136) umfassen, die konzipiert sind, um einen Wert ($P_{ref}^{(i)}{}_{F/B}$) der Fokussierungsparameter, die durch die vordefinierten Assoziationen assoziiert sind, an mindestens eine Position ($S^{(i)}$) auf dem gewünschten Verlauf ausgehend von der Position $P_{B/O}$ des Gehäuses (110) zu liefern.

4. Ultraschallsondiervorrichtung nach Anspruch 3, wobei die Aktualisierungsmittel (132) konzipiert sind, um die Fokussierungsparameter $P_{F/B}$ auf den Wert ($P_{ref}^{(i)}{}_{F/B}$) der Fokussierungsparameter, der von den Auswahlmitteln (114, 136) geliefert wird, zu aktualisieren.

5. Ultraschallsondiervorrichtung nach Anspruch 3, wobei die Aktualisierungsmittel (132) außerdem Folgendes umfassen:

    - Neueinstellmittel (114, 402), die konzipiert sind, um eine Position des Gehäuses (110) in Bezug zu dem Referenzwerk, das mit dem Objekt (102) verbunden ist, die auf dem gewünschten Verlauf ausgehend von der Position $P_{B/O}$ des Gehäuses (110) neu eingestellt ist, wobei diese neu eingestellte Position $\overline{P_{B/O}}$ genannt wird, und/oder eine Ausrichtung des Gehäuses (110), das auf eine gewünschte Ausrichtung des Gehäuses (110) ausgehend von der Ausrichtung $O_{B/O}$ des Gehäuses (110) neu eingestellt ist, wobei diese neu eingestellte Ausrichtung $\overline{O_{B/O}}$ genannt wird, zu bestimmen,
    - Mittel zur Abweichungsbestimmung (114, 406), die konzipiert sind, um eine Positionsabweichung, die $P_{B/B}$ genannt wird, zwischen der Position $P_{B/O}$ des Gehäuses (110) und der neu eingestellten Position $\overline{P_{B/O}}$ zu bestimmen, und/oder eine Ausrichtungsabweichung, die $O_{B/B}$ genannt wird, zwischen der Ausrichtung $O_{B/O}$ des Gehäuses (110) und der neu eingestellten Ausrichtung $\overline{O_{B/O}}$ zu bestimmen,
    - Korrekturmittel (114, 408), die konzipiert sind, um den Wert ($P_{ref}^{(i)}{}_{F/B}$) der Fokussierungsparameter, der von den Auswahlmitteln (136) geliefert wird, ausgehend von der Positionsabweichung $P_{B/B}$ und/oder der Ausrichtungsabweichung $\overline{O_{B/B}}$ zu korrigieren,

und wobei die Aktualisierungsmittel (132) konzipiert sind, um die Fokussierungsparameter $P_{F/B}$ auf den korrigierten Wert der Fokussierungsparameter, der von den Korrekturmitteln (114, 408) geliefert wird, zu aktualisieren.

6. Ultraschallsondiervorrichtung nach einem der Ansprüche 3 bis 5, wobei:

- der gewünschte Verlauf Verlaufssegmente $S^{(i)}$ umfasst und die vordefinierten Assoziationen Referenzwerte $P_{ref}^{(i)}{}_{F/B}$ der Fokussierungsparameter, und daher einer gewünschten Position des Brennpunkts in Bezug zu dem Referenzwerk, das mit dem Gehäuse verbunden ist, beziehungsweise mit jedem Verlaufssegment $S^{(i)}$ assoziieren,

- die Auswahlmittel (114, 136) konzipiert sind, um eines der Verlaufssegmente $S^{(i)}$ ausgehend von der Position $P_{B/O}$ des Gehäuses (110) auszuwählen, und um den Referenzwert $P_{ref}^{(i)}{}_{F/B}$, der mit dem ausgewählten Verlaufssegment $S^{(i)}$ assoziiert ist, zu liefern.

7. Ultraschallsondiervorrichtung nach einem der Ansprüche 3 bis 6, wobei die vordefinierten Assoziationen Parameter eines Neueinstellungsvorgangs umfassen, die einen Wert der Brennpunkte ausgehend von einer Position auf dem gewünschten Verlauf geben, und wobei die Auswahlmittel (114, 136) Mittel (114, 704) umfassen, die konzipiert sind, um mittels der Parameter des Neueinstellungsvorgangs den Wert der Fokussierungsparameter ausgehend von einer Position auf dem gewünschten Verlauf, der ausgehend von der Position $P_{B/O}$ des Gehäuses (110) bestimmt wird, zu berechnen.

8. Ultraschallsondiervorrichtung nach einem der Ansprüche 1 bis 7, wobei die Messgrößenumformer $(120_1 \dots 120_N)$ mobil an dem Gehäuse (110) angebracht sind, Ortungsmittel (122) der Messgrößenumformer $(120_1 \dots 120_N)$ aufweist, die konzipiert sind, um Positionen $P_{t/B}$ der Messgrößenumformer $(120_1 \dots 120_N)$ in Bezug zu einem Referenzwerk, das mit dem Gehäuse (110) verbunden ist, zu bestimmen, und wobei die Mittel (114, 138) zum Bestimmen des Gesetzes der Verzögerungen konzipiert sind, um das Gesetz der Verzögerungen außerdem ausgehend von diesen Positionen $P_{t/B}$ der Messgrößenumformer $(120_1 \dots 120_N)$ zu bestimmen.

9. Steuerverfahren von Messgrößenumformern $(120_1 \dots 120_N)$ einer Ultraschallsonde, das die folgenden Schritte umfasst:

- Empfangen (208) einer Position $P_{B/O}$ eines Gehäuses (110) der Sonde in Bezug zu einem Referenzwerk, das mit einem zu sondierenden Objekt (102) verbunden ist, wobei die Messgrößenumformer $(120_1 \dots 120_N)$ an diesem Gehäuse (110) angebracht sind,

- Bestimmen (222) eines Gesetzes der Verzögerungen (R) ausgehend von einer gewünschten Position eines Brennpunkts (F) in Bezug zu einem Referenzwerk, das mit dem Gehäuse (110) verbunden ist, wobei diese gewünschte Position durch Fokussierungsparameter mit der Bezeichnung $P_{F/B}$ dargestellt sind,

- Liefern (230) der Steuersignale (C) an die Messgrößenumformer $(120_1 \dots 120_N)$ ausgehend von dem Gesetz der Verzögerungen,

dadurch gekennzeichnet, dass es ferner Folgendes umfasst:

- einen Schritt (204) zum Definieren und Speichern:

-- vorbestimmter Positionen $(S^{(i)})$ auf einem gewünschten Verlauf des Gehäuses (110) in Bezug zu dem Referenzwerk, das mit dem Objekt (102) verbunden ist, und

-- vordefinierter Assoziationen mehrerer unterschiedlicher gewünschter Positionen $(P_{ref}^{(i)}{}_{F/B})$ des Brennpunkts (F) in Bezug zu dem Referenzwerk, das mit dem Gehäuse (110) verbunden ist, mit mehreren unterschiedlichen Positionen der vorbestimmten Positionen $(S^{(i)})$ auf dem gewünschten Verlauf,

- einen Schritt (210) für die Aktualisierung der gewünschten Position $(P_{ref}^{(i)}{}_{F/B})$ des Brennpunkts (F) in Bezug zu dem Referenzwerk, das mit dem Gehäuse verbunden ist, und daher der entsprechenden Fokussierungsparameter $P_{F/B}$, einerseits ausgehend von den gespeicherten vordefinierten Assoziationen und, andererseits, von der Position $P_{B/O}$ des Gehäuses (110), und

- einen Schritt (222) zur Aktualisierung des Gesetzes der Verzögerungen (R) ausgehend von der Aktualisierung der gewünschten Position des Brennpunkts (F) in Bezug zu dem Referenzwerk, das mit dem Gehäuse (110) verbunden ist, und der entsprechenden Fokussierungsparameter $P_{F/B}$.

10. Rechnerprogramm, das von einem Kommunikationsnetzwerk herunterladbar und/oder auf einem Träger aufgezeichnet ist, das durch einen Rechner gelesen werden und/oder von einem Prozessor ausgeführt werden kann, dadurch gekennzeichnet, dass es Anweisungen zur Umsetzung eines Steuerverfahrens von Messgrößenumformern einer Ultraschallsonde nach Anspruch 9 umfasst, wenn das Programm von einem Rechner ausgeführt wird.

**Claims**

1. Ultrasound probing device comprising:

   - a box (110),
   - locating means (130) designed to provide a position $P_{B/O}$ of the box (110) in relation to a reference frame associated with an object to be examined (102),
   - means (114, 138) for determining a delay law (R) on the basis of a desired position of a focal point (F) in relation to a reference frame associated with the box (110), wherein this desired position is represented by focusing parameters referenced $P_{F/B}$,
   - control means (114, 146) designed to provide control signals (C) on the basis of the delay law (R),
   - transducers ($120_1$...$120_N$) attached to the box (110), designed to receive control signals and, in response, to transmit ultrasonic waves in the object (102) which are delayed with respect to each other according to the delay law such that they focus at the focal point (F) defined by the focusing parameters $P_{F/B}$,

   **characterised in that**:

   - it comprises means (134) for storing:

     • predetermined positions ($S^{(i)}$) on a desired path of the box (110) in relation to the reference frame associated with the object (102), and
     • predefined associations of several different desired positions ($P_{ref}^{(i)}{}_{F/B}$) of the focal point (F) in relation to the reference frame associated with the box (110) at several different positions among said predetermined positions ($S^{(i)}$) on this desired path,

   - it further comprises means (132) for updating the desired position ($P_{ref}^{(i)}{}_{F/B}$) of the focal point (F) in relation to the reference frame associated with the box and thus the corresponding focusing parameters $P_{F/B}$ based, on the one hand, on said stored predefined associations and, on the other hand, on the position $P_{B/O}$ of the box (110), and
   - the means for determining (114, 138) are designed for an update of the delay law (R) based on the update of the desired positon of the focal point (F) in relation to the reference frame associated to the box (110) and on the corresponding focusing parameters $P_{F/B}$.

2. Ultrasound probing device according to claim 1, wherein the means (130) for locating the box (110) comprise a sensor designed to measure the position $P_{B/O}$ of the box (110).

3. Ultrasound probing device according to claim 1 or 2, wherein the updating means (132) comprise selection means (114, 136) designed to provide a focusing parameter value ($P_{ref}^{(i)}{}_{F/B}$) associated, by means of the predefined associations, with at least one position ($S^{(i)}$) on the desired path, on the basis of the position $P_{B/O}$ of the box (110).

4. Ultrasound probing device according to claim 3, wherein the updating means (132) are designed to update the focusing parameters $P_{F/B}$ to the focusing parameter value ($P_{ref}^{(i)}{}_{F/B}$) provided by the selection means (114, 136).

5. Ultrasound probing device according to claim 3, wherein the updating means (132) further comprise:

   - adjustment means (114, 402) designed to determine an adjusted position of the box (110) in relation to the reference frame associated with the object (102) on the desired path on the basis of the position $P_{B/O}$ of the box (110), this adjusted position being referenced $\overline{P_{B/O}}$, and/or an adjusted orientation of the box (110) on a desired orientation of the box (110) on the basis of the orientation $O_{B/O}$ of the box (110), this adjusted orientation being referenced $\overline{O_{B/O}}$,
   - means for determining deviations (114, 406) designed to determine a deviation in position, referenced $P_{B/B}$, between the position $P_{B/O}$ of the box (110) and the adjusted position $\overline{P_{B/O}}$, and/or a deviation in orientation, referenced $O_{B/B}$, between the orientation $O_{B/O}$ of the box (110) and the adjusted orientation $\overline{O_{B/O}}$,
   - correction means (114, 408) designed to correct the focusing parameter value ($P_{ref}^{(i)}{}_{F/B}$) provided by the selection means (136) on the basis of the deviation in position $P_{B/B}$ and/or the deviation in orientation $O_{B/B}$,

   and wherein the updating means (132) are designed to update the focusing parameters $P_{F/B}$ to the corrected focusing parameter value provided by the correction means (114, 408).

6. Ultrasound probing device according to any of claims 3 to 5, wherein:

   - the desired path comprising path segments $S^{(i)}$, the predefined associations associate reference values $P_{ref}^{(i)}{}_{F/B}$ of the focusing parameters and thus of a desired position of the focal point in relation to the reference frame associated with the box respectively with each path segment S$^{(i)}$,

- the selection means (114, 136) are designed to select one of the path segments S$^{(i)}$ on the basis of the position P$_{B/O}$ of the box (110) and to provide the reference value P$_{ref}^{(i)}$$_{F/B}$ associated with the selected path segment S$^{(i)}$.

**7.** Ultrasound probing device according to any of claims 3 to 6, wherein the predefined associations comprise parameters of an adjustment operation providing a focusing parameter value on the basis of a position on the desired path and the selection means (114, 136) comprise means (114, 704) designed to calculate, using the adjustment operation parameters, the value of the focusing parameters on the basis of a position on the desired path determined on the basis of the position P$_{B/O}$ of the box (110).

**8.** Ultrasound probing device according to any of claims 1 to 7, wherein the transducers (120$_1$...120$_N$) are attached in a movable manner to the box (110), comprising means (122) for locating the transducers (120$_1$...120$_N$) designed to determine positions P$_{t/B}$ of the transducers (120$_1$...120$_N$) in relation to a reference frame associated with the box (110), and wherein the means (114, 138) for determining the delay law are further designed to determine the delay law on the basis of these positions P$_{t/B}$ of the transducers (120$_1$...120$_N$).

**9.** Method for controlling the transducers (120$_1$...120$_N$) of an ultrasound probe, comprising the following steps:

- receiving (208) a position P$_{B/O}$ of a box (110) of the probe in relation to a reference frame associated with an object to be examined (102), the transducers (120$_1$...120$_N$) being attached to this box (110),
- determining (222) a delay law (R) on the basis of a desired position of a focal point (F) in relation to a reference frame associated with the box (110), wherein this desired position is represented by focusing parameters referenced P$_{F/B}$,
- providing (230) control signals (C) to the transducers (120$_1$...120$_N$) on the basis of the delay law,

**characterised in that** it further comprises:

- a step (204) for defining and storing:

    • predetermined positions (S$^{(i)}$) on a desired path of the box (110) in relation to the reference frame associated with the object (102), and
    • predefined associations of several different desired positions (P$_{ref}^{(i)}$$_{F/B}$) of the focal point (F) in relation to the reference frame associated with the box (110) at several different positions among said predetermined positions (S$^{(i)}$) on this desired path,

- a step (210) for updating the desired position (P$_{ref}^{(i)}$$_{F/B}$) of the focal point (F) in relation to the reference frame associated with the box and thus the corresponding focusing parameters P$_{F/B}$ based, on the one hand, on said stored predefined associations and, on the other hand, on the position P$_{B/O}$ of the box (110), and
- a step (222) for updating the delay law (R) based on the update of the desired positon of the focal point (F) in relation to the reference frame associated to the box (110) and on the corresponding focusing parameters P$_{F/B}$.

**10.** Computer program suitable for being downloaded from a communication network and/or saved on a medium suitable for being read by a computer and/or executed by a processor, comprising instructions for the implementation of a method for controlling transducers of an ultrasound probe according to the invention, when said program is executed by a computer.

<u>*Figure 1*</u>

*100*

*116*

*132*

*119*

*134*

$P_{ref}^{(i)}{}_{F/B} <-> S^{(i)}$

M

*112*

*118*

*146* — R — *144* — $P_{F/O}$ — *142* — $P_{F/B}$ — $P_{ref}^{(i)}{}_{F/B}$ — *136*

C

*114*

M

$P_{t/O}$

C

*128* — $P_{t/B}$ — *140*

*138*

$P_{B/O}$

*122*

*104*

*108*

*106*

*130*

$124_1...124_N$

*126*

*110*

$120_1...120_N$

*102*

$P_{F/B}$

F

Figure 2

Figure 5

## Figure 3

## Figure 6

## Figure 8

## Figure 4

Figure 7

**EP 2 628 000 B1**

## RÉFÉRENCES CITÉES DANS LA DESCRIPTION

**Documents brevets cités dans la description**

- FR 2786651